Europäisches Patentamt

European Patent Office

Office européen des brevets

(19)

(11) Publication number: **0 126 814**
**B1**

# EUROPEAN PATENT SPECIFICATION

(12)

(45) Date of publication of patent specification: **21.12.88**

(51) Int. Cl.⁴: **A 61 B 17/39**

(21) Application number: **83302992.9**

(22) Date of filing: **24.05.83**

(54) Electro-surgical unit control apparatus.

(43) Date of publication of application:
**05.12.84 Bulletin 84/49**

(45) Publication of the grant of the patent:
**21.12.88 Bulletin 88/51**

(84) Designated Contracting States:
**CH DE FR GB IT LI**

(56) References cited:
**DE-A-3 045 996**
**FR-A-2 502 935**
**GB-A-1 498 338**

(73) Proprietor: **Chang, Sien-Shih**
**1st Fl., No. 171 East Division**
**Veterans General Hospital Taipei (TW)**
(73) Proprietor: **Young, Shuenn-Tsong**
**3rd Fl., No. 46 Lane 45, Sec. 1 Min Deng Rd.**
**Ruey Fang Town Taipei (TW)**

(72) Inventor: **Chang, Sien-Shih**
**1st Fl., No. 171 East Division**
**Veterans General Hospital Taipei (TW)**
Inventor: **Young, Shuenn-Tsong**
**3rd Fl., No. 46 Lane 45, Sec. 1 Min Deng Rd.**
**Ruey Fang Town Taipei (TW)**

(74) Representative: **Baillie, Iain Cameron et al**
**c/o Ladas & Parry Isartorplatz 5**
**D-8000 München 2 (DE)**

Courier Press, Leamington Spa, England.

EP 0 126 814 B1

# Description

This invention relates to a control apparatus for an electrosurgical unit (E.S.U.) control apparatus.

An E.S.U. such as that manufactured by Electro Medical System under type No. 2000DP is used in performing a transurethral resection (T.U.R.) and with present apparatus it takes approximately four years to train a surgeon to perform a T.U.R. In the T.U.R. operation the E.S.U. is connected to an active electrode cutting device formed by a NiCr loop.

It is an object of this invention to provide an apparatus for controlling the output current or power of the E.S.U. to control the cutting action of the active electrode cutting device and to thereby render the operation procedure safer and to assist the surgeon.

According to this invention there is provided an electro-surgical control apparatus adapted to perform an transurethral resection comprising an electro-surgical unit and an electrical circuit formed therewith by an active electrode cutting device formed by a NiCr loop, an electrode plate and a return connection from said electrode plate to said electro-surgical unit characterized by an electro-magnetic sensing means arranged to detect the output current or power from the electro-surgical unit which, in operation, maintains a constant output voltage, electrically operated switch means in the electrical path from the electro-surgical unit to the cutting device, and comparison means connected to receive an input from the sensing means and arranged to compare the said received input with stored data representative of different types of tissue and to provide an output signal to said switch means in dependence upon the status of the comparison, whereby the switch means is arranged to open-circuit the path from the electro-surgical unit to the cutting device upon a predetermined threshold being reached by said received input.

Preferably the sensing means is a current sensing probe and conveniently the switch means is an electromagnetic relay.

An audible alarm may also be provided for actuation upon said predetermined threshold being reached.

Preferably the comparison means includes a central processing unit connected with a read only memory which is arranged to store the control steps of the central processing unit and a random access memory arranged to act as a working store for the central processing unit.

Conveniently the stored data held by the comparison means is inputted thereto by a keyboard and advantageously a display unit is provided for displaying the data inputted by the keyboard or the result of comparison by the central processing unit.

In a preferred embodiment the current sensing probe is connected to a peak detector comprising operational amplifiers for detecting the positive and negative peaks of the sensed alternating signal and a summing amplifier for summing the outputs of the said operational amplifiers.

In said preferred embodiment the output of the summing amplifier is arranged to be applied to a phase shifter and thence to one input of a comparator, the other input of the comparator being connected to receive the non-shifted output of the summing amplifier, the output of the comparator and of the peak detector being connected to a sample and hold circuit for generating a control signal which after analog/digital conversion is applied to the central processing unit for comparison with the stored data.

The present invention is based upon the fact that the adenoma consists of a great amount of electrolyte and is an irregular soft and fluffy tissue which exhibits a low resistance. In distinction the capsule has little electrolyte and is formed by netted fibrous tissue having a relatively high resistance. Thus when active electrode cutting device touches the adenoma the output power from the E.S.U. and current is increased and this increased current is detected by the sensing probe whereas when the active electrode cutting device touches the capsule the output current from the E.S.U. is low and by setting stored data into the control apparatus of this invention the output current of the E.S.U. may be cut off and an alarm circuit may be energised when the cutting device touches the capsule.

The apparatus is therefore used to determine the differences in conductivity in the prostate gland which cause the load presented to the E.S.U. to vary during T.U.R. operating procedure and to cut off the output current or power of the E.S.U. when the cutting device touches the capsule.

The invention will now be described by way of example with reference to the accompanying drawings in which:—

Figure 1 shows a simplified sectional view of a prostate gland;

Figure 2 shows the apparatus in accordance with the invention connected in a circuit utilising an E.S.U.;

Figure 3 shows a block diagram of the apparatus;

Figure 4 shows a circuit diagram of the peak detector and summing circuit shown in Figure 3;

Figure 5 shows a circuit diagram of the phase shifter and comparator shown in Fig. 3;

Figure 6 shows a circuit diagram of the sample/hold circuit and Analog/Digital converter shown in Figure 3;

Figure 7 shows a circuit diagram of the parallel input/output circuit;

Figure 8 shows a circuit diagram of an interface circuit with the alarm and relay shown in Figure 3;

Figure 9 shows a circuit diagram of the CPU, ROM, RAM and Clock Generator of Figure 3;

Figure 10 shows a circuit diagram of the keyboard/display controller, driver and display;

Figure 11 is a flow chart of the control program, and

Figures 12 and 13 shows overall circuit diagrams of the control apparatus.

In the Figures like references denote like numerals.

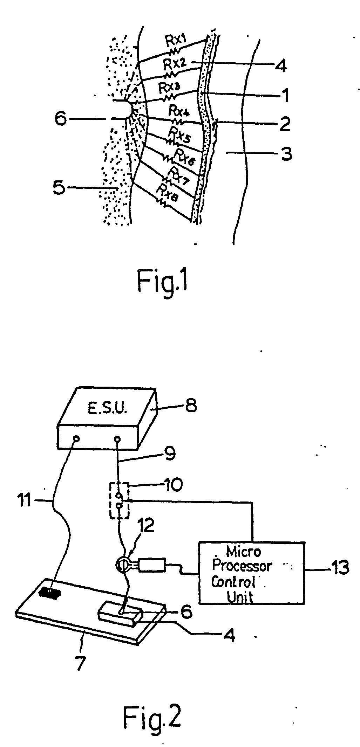

Referring to Figure 1, a simplified cross-section of a prostate gland is shown. The prostate gland has an outer fibroelastic capsule 1 which is formed by netted fibrous tissue and which contains a little electrolyte. On the outer surface of the capsule 1 is fat 2 which is covered by tissue 3. On the inner surface of the capsule 1 is the adenoma 4 which is formed by an irregular, soft and fluffy tissue containing a large amount of electrolyte and which may be considered as an infinite plurality of parallel resistances RX1—RX8. On the inside surface of the adenoma 4 is distilled water 5 and an active electrode cutting device formed by a NiCr cutting loop 6 is shown in the region of distilled water (electrolyte) 5 and being linked with one end of each of the parallel resistors RX1—RX8 as shown by broken lines.

As shown in Figures 1 and 2 the NiCr cutting loop 6 is positioned inside the prostate gland and the patient lies on an electrode plate 7 in a position such that the adenoma 4 forms a load to an electro-surgical unit 8 which is connected to the NiCr cutting loop 6 by a lead 9 in which is positioned a relay 10. A return path from the electrode plate 7 to the E.S.U. is formed by a return wire 11. A current probe 12 is positioned about the wire 9 and connected to a control unit 13.

In operation the voltage of the E.S.U. is maintained constant so that the output power and hence current of the E.S.U. varies according to the load into which the NiCr cutting loop 6 is in contact. The different tissues and different inclusions in the prostate gland have differing consistency with a result that the conductivity thereof will vary. The resistance of the compact fibroelastic capsule 1 and the fat 2 which both contain little electrolyte is high while the resistance of the loose, irregular and fluffy tissue of the adenoma 4 contains a large amount of electrolyte and the resistance thereof is low. Accordingly, the differencies in conductivity of the load to the E.S.U. vary during the operating procedure of T.U.R. Thus the tissue resected can be distinguished according to the output power or current of the E.S.U.

The E.S.U. typically provides an output current at a frequency of 550 kHz so that with the NiCr loop 6 inside the prostate gland merging in the adenoma 4, the resistance between the NiCr loop 6 and the adenoma 4 is low since the adenoma may be considered as an unlimited number of equivalent resistances in parallel which, according to Ohm's Law reduce the total resistance since the resistors are in parallel. Thus in the low resistance, high conductivity region of the adenoma 4 the output power and hence current of the E.S.U. is high. In distinction the output power of the E.S.U. and hence output current will be low in the higher resistance, low conductivity region of the capsule 1. The sensor 12 is thus able to sense the current drawn by the NiCr cutting loop so that when the NiCr loop 6 touches the high resistance, low conductivity capsule the control unit 13 is arranged to turn on an audible alarm and to cut off by opening relay 10. Thus with the control apparatus of the present invention the safety of the patient is increased during a T.U.R. operation and the time for training a surgeon decreases.

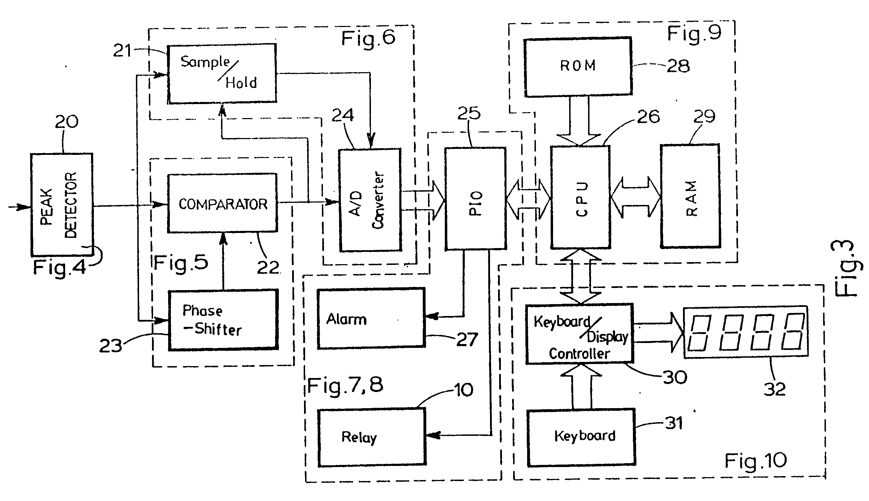

The control unit 13 will now be described with reference to Figures 3—13 of which Figure 3 is an overall schematic of the unit. Input from the current probe 12 (not shown in Figure 3) is applied to a peak detector 20 which applies an output to a sample and hold circuit 21, a comparator 22 and a phase shifter 23. The phase shifter is used to shift the peak input current phase by minus 5°—10° and applies the phase shifted signal to a further input of the comparator 22 so that the comparator 22 compares the shifted and non-shifted peak signal input. The output from the comparator is fed to a further input of the sample and hold circuit 21 and to an analog/digital (A/D) convertor 24 which is also arranged to receive at a further input terminal output from the sample and hold circuit 21. The sample and hold circuit 21 is employed to hold data stable for use by the Central Processing Unit (CPU) to be described herein later. The A/D convertor converts the analog signal to a digital form and applies an output signal to parallel input/output (PIO) circuit 25 which interfaces with a CPU 26 and peripheral devices formed by an audible alarm circuit 27 and the relay 10. The CPU 26 is arranged to execute logical operations, process and analyse data for controlling the necessary devices and reads out instructions from a read only memory (ROM) 28 and is connected via a 2-way highway to a random access memory (RAM) 29. The ROM 28 stores the fixed control sequence to be performed by the CPU and the RAM is a working store for temporary data upon which the CPU operates. The CPU 26 is also connected over a 2-way highway to a keyboard/display controller 30 which receives input from a keyboard 31 and provides output to a display 32. The controller 30 is arranged to scan the keyboard and display and encode binary data for CPU use and to encode the binary data to drive the seven segment display 32. The keyboard is provided for inputting parameters to the microprocessor and the display 32 displays the parameters.

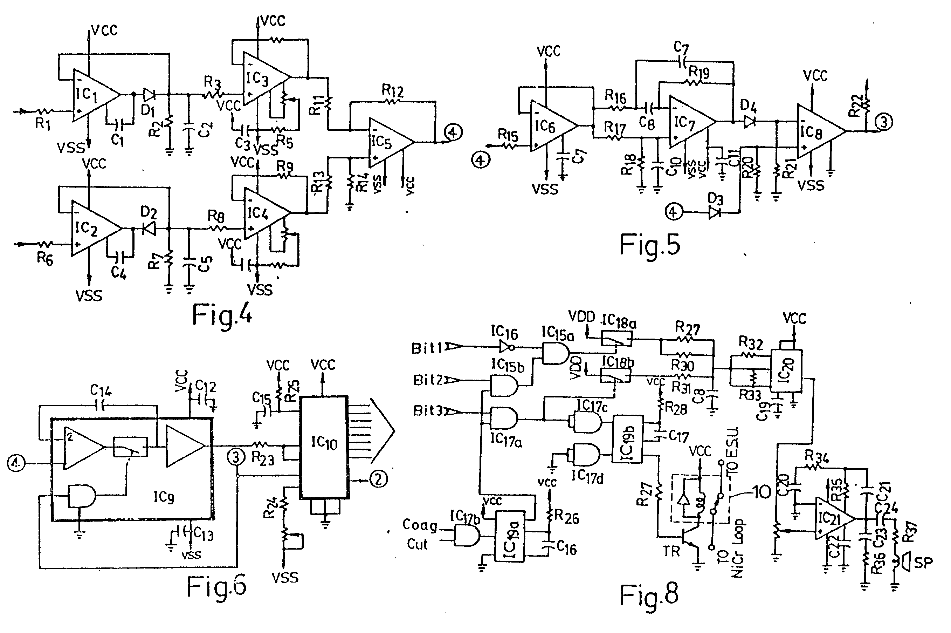

The various circuit elements shown in Figure 3 are shown in greater detail in Figures 4—10 and because these circuits are reproduced in the figures with particularity it is not thought necessary that these figures need be discussed in detail since the construction and operation thereof will be self evident to the man skilled in the art. Thus these figures will only briefly be discussed.

Referring to Figure 4 the circuit of the peak detector 20 is shown which includes five oper-

ational amplifiers IC1—IC5. The operational amplifiers IC1 and IC2 act as buffers having very high impedance to increase the signal/noise ratio and are connected so that the positive signal picked up by the sensor probe 12 passes through IC1 and the negative signal passes through IC2 with IC3 and IC4 amplifying these signals. The outputs of IC3 and IC4 are summed by IC5 to provide a summed output signal.

Figure 5 shows the detailed circuit of the comparator 22 and phase shifter 23 and includes three integrated circuits IC6—IC8. Integrated circuit IC6 acts as a voltage follower receiving input from IC5 and is used to effectively isolate the input signal prior to being shifted in phase by integrated circuit IC7. The phase shifter IC7 and its associated components R16—R19 and C8—C10 is used to shift the input signal by minus 5° to 10° and to feed the phase lagging signal to the inverting input of integrated circuit IC8 which acts as a comparator. The non-inverting input of the comparator IC8 receives the non-shifted phase signal from the output of amplifier IC5. Thus comparator IC8 compares the non-shifted input signal with the phase shifted signal and each time the peak signals coincide a control pulse is generated to control the sample/hold circuit and the A/D convertor IC10, both shown in Figure 6.

One input of the sample/hold circuit IC9 receives input from the output of amplifier IC5 and another input thereof receives input from the output of comparator IC8 which latter output is also applied to the input of the A/D convertor IC10 and the A/D convertor IC10 also receives at an input output from the sample/hold circuit IC9. Pin 2 of the sample/hold circuit IC9 is a sampling control pin providing output to a capacitor C14 which holds the sampling signal for a few milliseconds to maintain a stable condition. A/D convertor IC10 then converts the stable analog signal to an eight bit binary signal for CPU use. Thus the comparator IC8 feeds its output control signal to control the sample and hold ciruit IC9 for input stabilisation and transmission to the A/D convertor input so as to control the data conversion of the A/D convertor.

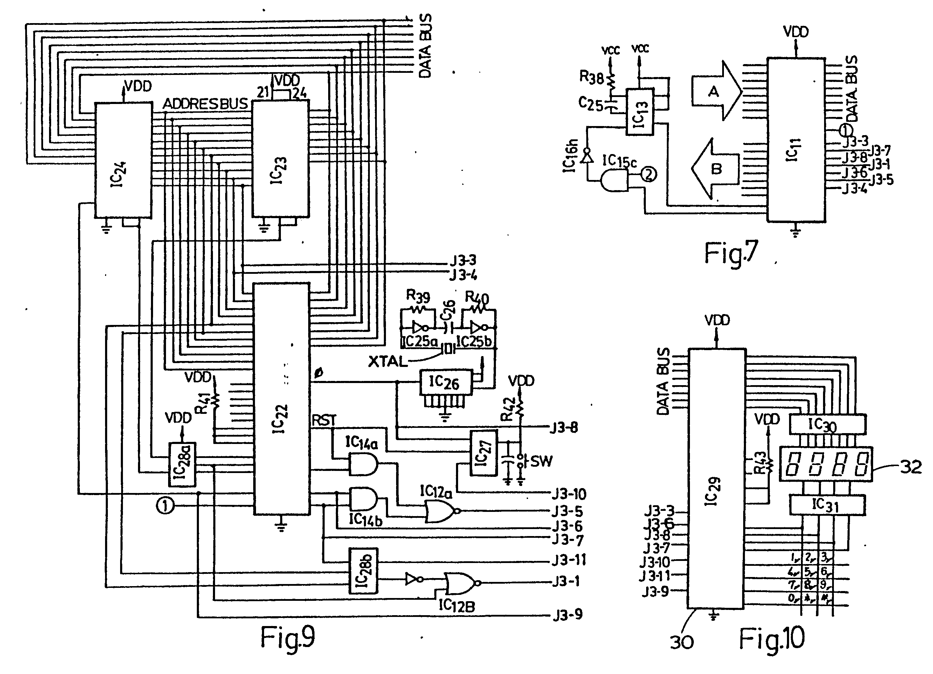

Figure 7 shows in detail the parallel input/ output circuit (PIO) which includes two input ports A and B. The port A transfers the data from the A/D convertor to the CPU 26 and port B latches the data and controls ON/OFF of the output power of the E.S.U. by virtue of relay 10 and the ON/OFF of the alarm circuit 27.

Figure 8 shows an interfacing circuit which includes integrated circuits IC15—IC21. Integrated circuit IC17 ANDS a coagulation signal and a cutting signal which are two control signal outputs from the E.S.U. and these two signals are synchronised with the output bits of the CPU to control the ON/OFF of the relay. Bits 2, 3 of the CPU data bus form a control signal and enable the relay 10 and an alarm circuit 27. An analog switch IC18a, IC18b is controlled by the CPU and the coagulation and cutting signals to generate two tones. Integrated circuit IC19b increases the width of the signal produced by circuit IC17a to permit the relay 10 to receive a sufficiently long pulse to effect correct operation. Integrated circuit IC20 for the alarm outputs a signal to an audio amplifier IC21 to drive a speaker SP. Integrated circuit IC19b controls the relay 10 whereby output power of the E.S.U. is controlled.

Referring to Figure 9, a clock generator performed by IC25a, IC25b resistors R39, R40, capacitor C26 and a crystal (XTAL) provide clock pulses which are divided by IC26 for use by the CPU IC22. The controlling sequence for the CPU is stored in a control programme of a ROM IC23 and temporary data is stored in RAM IC24.

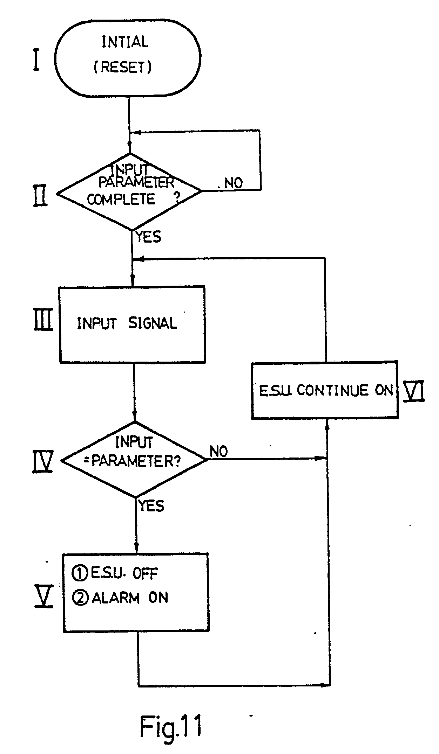

Integrated circuit IC29 shown in Figure 10 receives input from the output of the CPU and is designed for keyboard scanning, data encoding and display scanning. Integrated circuits IC30 and IC31 serve as current drivers for the seven segment display 32 which is formed by light emitting diodes (LED'S). The seven segment display 32 is provided to display the parameters in the CPU or display the program stored in the ROM or data stored in the RAM.

The ROM control programme is shown in Figure 11 where step I resets the CPU and starts the operation of the apparatus. Step II checks to determine that the inputs from the keyboard have been correctly inputted. If an affirmative answer is provided then the input signal is continuously sampled at step III but if a negative answer is provided at step II then the sequence is returned to the input of step II. The output of the sampling is applied at step IV where equivalence with predeterminedly stored parameters is effected. In this respect the predeterminedly stored parameters inputted by the keyboard may be indictive of the NiCr loop touching the capsule and if such equivalence is found then output is applied to step V to turn the alarm circuit ON and to cut the power supply OFF by opening the relay 10. If a negative response is found at step IV, or alternatively upon completion of the step V, a signal is passed to step VI to continue applying power from the E.S.U. to the NiCr cutting loop.

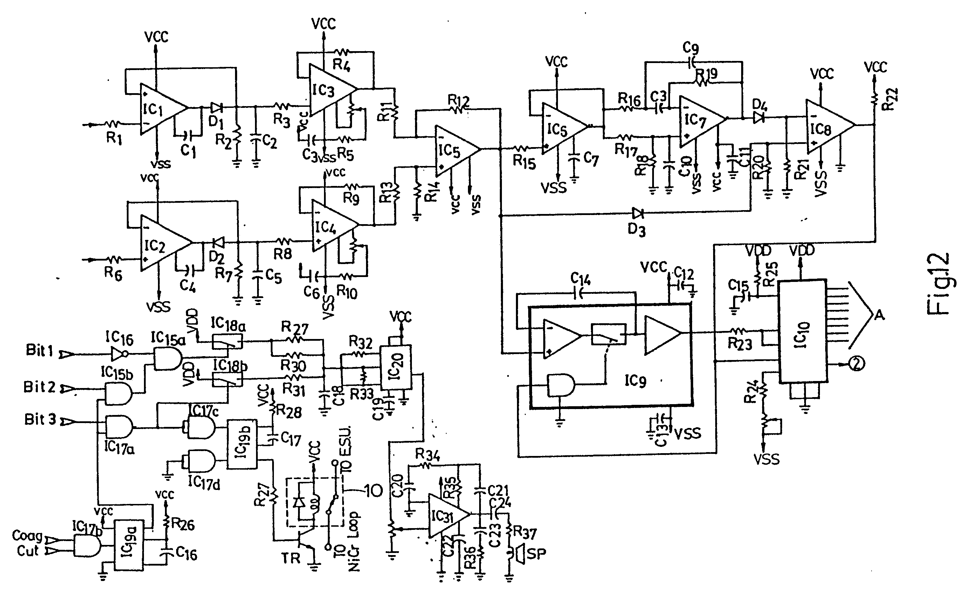

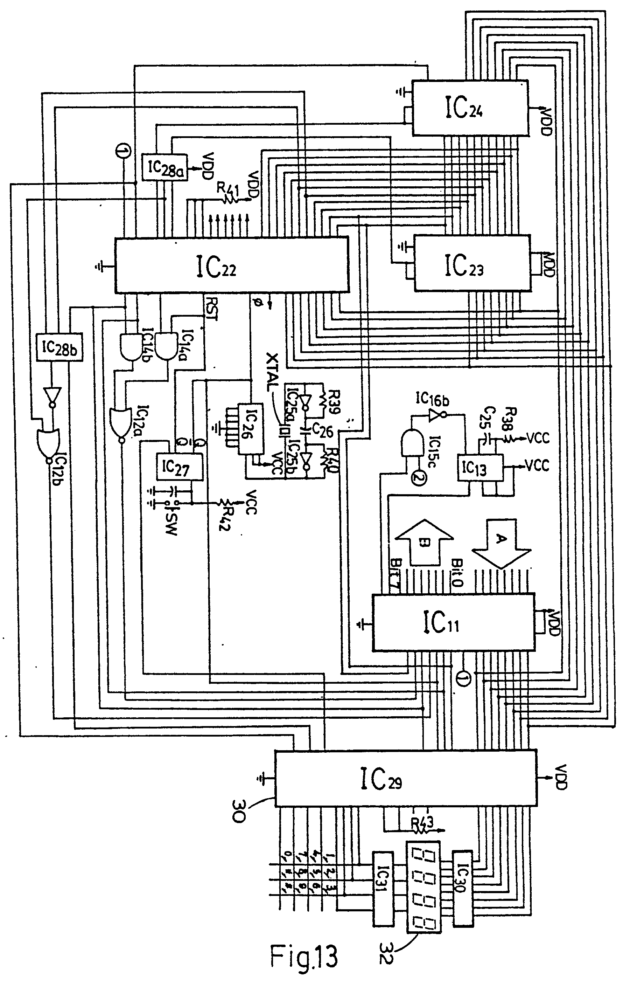

The overall circuit diagram is shown in Figures 12 and 13 and shows the interconnections of the various Figures 4—10.

Thus it will now be appreciated that by the use of the present invention to sense current flow from an E.S.U. to a NiCr loop as described in the above preferred embodiment or, alternatively, by sensing the output power of the E.S.U. so, because the current or power varies in accordance with the load placed upon the NiCr loop so vitally important information is provided to a surgeon which by cutting off the power from the E.S.U. is able to prevent a possibly serious mistake being made by the surgeon.

The integrated circuits described herein are commercially available products from National Semiconductor, RCA, Texas Instruments, T.R.W. and Raytheon.

## Claims

1. An electro-surgical control apparatus adapted to perform a transurethral resection comprising an electro-surgical unit (8) and an electrical circuit formed therewith by an active electrode cutting device formed by a NiCr loop (6), an electrode plate (7) and a return connection (11) from said electrode plate to said electro-surgical unit characterized by an electro-magnetic sensing means (12) arranged to detect the output current or power from the electro-surgical unit (8) which, in operation, maintains a constant output voltage, electrically operated switch means (10) in the electrical path from the electro-surgical unit to the cutting device (6), and comparison means (13) connected to receive an input from the sensing means and arranged to compare the said received input with stored data representative of different types of tissue and to provide an output signal to said switch means (10) in dependence upon the status of the comparison, whereby the switch means (10) is arranged to open-circuit the path from the electro-surgical unit (8) to the cutting device (6) upon a predetermined threshold being reached by said received input.

2. An electro-surgical control apparatus as claimed in claim 1 characterized in that the sensing means is a current sensing probe (12).

3. An electro-surgical control apparatus as claimed in claim 1 or 2 characterized in that the switch means is an electromagnetic relay (10).

4. An electro-surgical control apparatus as claimed in any preceding claim characterized in that an audible alarm (27) is provided for actuation upon said predetermined threshold being reached.

5. An electro-surgical control apparatus as claimed in any preceding claim characterized in that the comparison means includes a central processing unit (26) connected with a read only memory (28) which is arranged to store the control steps of the central processing unit and a random access memory (29) arranged to act as a working store for the central processing unit.

6. An electro-surgical control apparatus as claimed in any preceding claim characterized in that the stored data held by the comparison means is inputted thereto by a keyboard (31).

7. An electro-surgical control apparatus as claimed in claim 6 characterized in that a display unit (32) is provided for displaying the data inputted by the keyboard or the result of comparison by the central processing unit.

8. An electro-surgical control apparatus as claimed in claim 2 characterized in that the current sensing probe is connected to a peak detector (20) comprising operational amplifiers (IC1—IC4) for detecting the positive and negative peaks of the sensed alternating signal and a summing amplifier (IC5) for summing the outputs of the said operational amplifiers.

9. An electro-surgical control apparatus as claimed in claim 8 characterized in that the output of the summing amplifier (IC5) is arranged to be applied to a phase shifter (23) and thence to one input of a comparator (22), the other input of the comparator being connected to receive the non-shifted output of the summing amplifier, the output of the comparator and of the peak detector being connected to a sample and hold circuit (21) for generating a control signal which after analog/digital conversion is applied to the central processing unit for comparison with the stored data.

## Patentansprüche

1. Eine elektro-chirurgische Steuervorrichtung zum Durchführen einer transurethralen Resektion, mit einer elektro-chirurgischen Einheit (8) und einer elektrischen Schaltung, die damit durch eine aktive Elektrodenschneidvorrichtung aus einer NiCr-Schlaufe (6) ausgebildet wird, mit einer Elektrodenplatte (7) und mit einer Rückführverbindung (11) von der Elektrodenplatte zu der elektro-chirurgischen Einheit, gekennzeichnet durch eine elektromagnetische Fühleinrichtung (12), die dazu eingerichtet ist, den Ausgangsstrom oder die Ausgangsleistung von der elektro-chirurgischen Einheit (8) abzufühlen, die im Betrieb eine konstante Ausgangsspannung aufrechterhält, durch elektrisch betätigte Schalteinrichtungen (10) im elektrischen Pfad von der elektrochirurgischen Einheit zu der Schneidvorrichtung (6), und durch Vergleichseinrichtungen (13), die verbunden sind, um ein Eingangssignal von der Fühleinrichtung zu empfangen, und die dazu eingerichtet sind, um das vorgenannte empfangene Eingangssignal mit abgespeicherten Daten zu vergleichen, die repräsentativ sind für verschiedene Arten von Gewebe, und um ein Ausgangssignal für die Schalteinrichtung (10) in Abhängigkeit von dem Status des Vergleiches zu erzeugen, wodurch die Schalteinrichtung (10) veranlaßt wird, aufgrund des Erreichens eines vorbestimmten Schwellwertes durch das vorgenannte empfangene Eingangssignal den Pfad von der elektro-chirurgischen Einheit (8) zu der Schneidvorrichtung (6) zu öffnen.

2. Eine elektro-chirurgische Steuervorrichtung nach Anspruch 1, dadurch gekennzeichnet, daß die Fühleinrichtung eine Stromfühlsonde (12) ist.

3. Eine elektro-chirurgische Steuervorrichtung nach Anspruch 1 oder 2, dadurch gekennzeichnet, daß die Schalteinrichtung ein elektromagnetisches Relais (10) ist.

4. Eine elektro-chirurgische Steuervorrichtung nach einem der voranstehenden Ansprüche, dadurch gekennzeichnet, daß ein hörbarer Alarm (27) vorgesehen ist zur Betätigung beim Erreichen des vorgenannten vorbestimmten Schwellwertes.

5. Eine elektro-chirurgische Steuervorrichtung nach einem der voranstehenden Ansprüche, dadurch gekennzeichnet, daß die Vergleichseinrichtung eine zentrale Verarbeitungseinheit (26) einschließt, die mit einem frei adressierbaren Festspeicher (ROM) (28), der vorgesehen ist, um die Steuerschritte der Zentraleinheit (CPU) zu speichern, und einem Direktzugriffsspeicher (RAM) (29) verbunden ist, der vorgesehen ist, um als der Arbeitsspeicher für die Zentraleinheit zu dienen.

6. Eine elektro-chirurgische Steuervorrichtung nach einem der voranstehenden Ansprüche, dadurch gekennzeichnet, daß die von der Vergleichseinrichtung bereitgehaltenen gespeicherten Daten durch ein Tastenfeld (31) dort eingegeben werden.

7. Eine elektro-chirurgische Steuervorrichtung nach Anspruch 6, dadurch gekennzeichnet, daß eine Bildschirmeinheit (32) vorgesehen ist zum Darstellen der durch das Tastenfeld eingegebenen Daten oder zum Darstellen des Ergebnisses des Vergleichs durch die Zentraleinheit.

8. Eine elektro-chirurgische Steuervorrichtung nach Anspruch 2, dadurch gekennzeichnet, daß die Stromfühlsonde verbunden ist mit einem Spitzenwertdetektor (20), der Operationsverstärker (IC1—C4) zum Finden der positiven und negativen Spitzen des abgefühlten alternierenden Signals und einen Summierverstärker (IC5) zum Aufsummieren der Ausgangssignale des vorgenannten Operationsverstärkers umfaßt.

9. Eine elektro-chirurgische Steuervorrichtung nach Anspruch 8, dadurch gekennzeichnet, daß der Ausgang des Summierverstärkers (IC5) auf einen Phasenschieber (23) und dann auf einen Eingang eines Vergleichers (22) gegeben werden kann, wobei der andere Eingang des Vergleichers angeschlossen wird, um den nicht phasenverschobenen Ausgang des Summierverstärkers zu empfangen, wobei der Ausgang des Vergleichers und des Spitzenwertdetektors verbunden ist mit einem Abtast- und Haltekreis (21) zum Erzeugen eines Steuersignals, welches nach einer analog/digital-Umwandlung auf die Zentraleinheit zum Vergleich mit den gespeicherten Daten gegeben wird.

**Revendications**

1. Appareil de commande en chirurgie électrique adapté pour réaliser une résection transurétrale, comprenant une unité de chirurgie électrique (8) et un circuit électrique comportant un dispositif de sectionnement (6) à électrode active, constitué par une boucle en NiCr, une plaque électrode (7) et une liaison de retour (11) reliant ladite plaque électrode à ladite unité de chirurgie électrique, caractérisé en ce qu'un organe de détection électromagnétique (12) est prévu pour détecter le courant ou la puissance de sortie de l'unité de chirurgie électrique (8) qui, en fonctionnement, maintient une tension de sortie constante, des moyens de commutation électriquement commandés, interposés dans la liaison électrique reliant l'unité de chirurgie électrique au dispositif de sectionnement, des moyens de comparaison (13) connectés pour recevoir un signal d'entrée provenant de l'organe de détection et réalisés de façon à comparer ledit signal d'entrée avec une information stockée et représentative des différents types de tissus et à délivrer un

signal de sortie auxdits moyens de commutation (10) en fonction de l'état de comparaison, de telle sorte que les moyens de commutation (10) puissent ouvrir le circuit entre l'unité de chirurgie électrique (8) et le dispositif de sectionnement (6) dès qu'un seuil prédéterminé est atteint par ledit signal d'entrée reçu.

2. Appareil de chirurgie électrique selon la revendication 1, caractérisé en ce que l'organe de détection est une sonde de détection de courant.

3. Appareil de chirurgie électrique selon l'une des revendications 1 ou 2, caractérisé en ce que les moyens de commutation sont constitués par un relai électromagnétique (10).

4. Appareil de chirurgie électrique selon l'une des revendications précédentes, caractérisé en ce qu'une alarme sonore (27) est prévue pour être actionnée dès que ledit seuil prédéterminé est atteint.

5. Appareil de chirurgie électrique selon l'une des revendications précédentes, caractérisé en ce que les moyens de comparaison comprennent une unité centrale de traitement (26) connectée à une mémoire de lecture seule (28) qui est adaptée pour stocker les étapes de commande de l'unité de traitement centrale et une mémoire d'accès aléatoire (29) qui est adaptée pour agir comme mémoire de travail pour l'unité de traitement centrale.

6. Appareil de chirurgie électrique selon l'une des revendications précédentes, caractérisé en ce que l'information stockée traitée par les moyens de comparaison est introduite dans ces derniers au moyen d'un clavier (31).

7. Appareil de chirurgie électrique selon la revendication 6, caractérisé en ce qu'une unité d'affichage (32) est prévue pour afficher l'information introduite au moyen du clavier ou le résultat de la comparaison de l'unité de traitement centrale.

8. Appareil de chirurgie électrique selon la revendication 2, caractérisé en ce que la sonde de détection de courant est connectée à une détecteur de pic (20) comportant des amplificateurs opérationnels (IC$_1$—IC$_4$) pour la détection des pics positif et négatif du signal alterné détecté et un amplificiateur de sommation (IC$_5$) pour sommer les sorties desdits amplificateurs opérationnels.

9. Appareil de chirurgie électrique selon la revendication 8, caractérisé en ce que la sortie de l'amplificateur de sommation (IC$_5$) est prévue pour être appliquée à un décaleur de phase (23) et de là à une entrée d'un comparateur (22), l'autre entrée du comparateur étant connectée pour recevoir la sortie non décalée de l'amplificateur de sommation, les sorties du comparateur et du détecteur de pic étant connectées à un circuit d'interrogation et de mémoire (21) pour générer un signal de commande qui, après conversion analog/digital, est appliqué à l'unité de traitement centrale pour comparaison avec l'information stockée.

Fig.1

Fig.2

Fig.3

Fig.4 — PEAK DETECTOR 20

Fig.5 — Phase-Shifter 23 — COMPARATOR 22

Fig.6 — Sample/Hold 21 — A/D Converter 24

Fig.7,8 — Relay 10 — Alarm 27 — PIO 25

Fig.9 — CPU 26 — ROM 28 — RAM 29

Fig.10 — Keyboard 31 — Keyboard/Display Controller 30 — 32

2

Fig.5

Fig.8

Fig.4

Fig.6

Fig.7

Fig.9

Fig.10

Fig.11

Fig.12

Fig.13